# EUROPÄISCHE PATENTSCHRIFT

(11) **EP 1 570 795 B1**
(45) Veröffentlichungstag und Bekanntmachung des Hinweises auf die Patenterteilung: **06.08.2008**
(21) Anmeldenummer: 05003401.6
(22) Anmeldetag: 17.02.2005
(51) Int. Cl.: A61B 17/70, A61B 17/64

(54) **Einrichtung zur dynamischen Stabilisierung von Wirbeln oder Knochen und stabförmiges Element dafür**
Apparatus for dynamic stabilisation of the spine or bones and rod-like element for same
Dispositif pour stabilisation dynamique du rachis ou des os et élément en forme de tige pour ce dispositif

(30) Priorität: 05.03.2004 DE 102004010844; 05.03.2004 US 550697 P
(43) Veröffentlichungstag der Anmeldung: 07.09.2005
(60) Teilanmeldung: 08009018.6
(73) Patentinhaber: BIEDERMANN MOTECH GmbH, 78054 VS-Schwenningen (DE)
(72) Erfinder: Harms, Jürgen, 76227 Karlsruhe (DE); Biedermann, Lutz, 78084 VS-Villingen (DE); Matthis, Wilfried, 79367 Weisweil (DE)
(74) Vertreter: Hofer, Dorothea

(56) Entgegenhaltungen:
- EP-A- 0 667 127
- WO-A-02/102259
- US-A- 5 415 661
- US-A1- 2003 191 470
- US-A1- 2004 015 166

## Beschreibung

Die Erfindung betrifft eine Stabilisierungseinrichtung zur dynamischen Stabilisierung von Wirbeln oder Knochen und ein stabförmiges Element für eine derartige Stabilisierungseinrichtung.

Aus der EP 1 188 416 A1 ist eine dynamische Stabilisierungseinrichtung zum Stabilisieren benachbarter Rückenwirbel bekannt, die zwei Pedikelschrauben und ein kabelartiges Band umfasst, welches in den Aufnahmeteilen der Pedikelschrauben jeweils über eine Klemmschraube befestigt ist und welche ein auf das Band aufgezogenes Stützelement in Form eines druckfesten Körpers beinhaltet.

Aus der US 2003/0109880 A1 ist eine dynamische Stabilisierungseinrichtung für Wirbel bekannt, die eine erste und eine zweite in einem Wirbel zu verankernde Schraube jeweils mit einem Aufnahmeteil zum Einlegen einer die Schrauben verbindenden Feder und eine solche Feder umfasst.

Aus der US 5,415,661 und der US 2003/0191470 A1 ist jeweils eine Stabilisierungseinrichtung für die Wirbelsäule bekannt, die einen gekrümmten elastischen Stab aufweist, der an seinen Enden über Verankerungselemente mit einem Schaft in den Fortsätzen benachbarter Wirbeln auf derselben Seite so verankert wird, daß der gekrümmte Stab und die Schäfte im wesentlichen in zueinander parallelen Ebenen liegen. Dadurch liegt die Krümmung in der anterioren/posterioren Richtung. Die Krümmung erfüllt die Funktion eines Federstabs.

Bei den bekannten Stabilisierungseinrichtungen werden jeweils zwei dieser Stabilisierungseinrichtungen zum Stabilisieren von zwei benachbarten Wirbeln verwendet, die jeweils rechts bzw. links von der Mittellängsachse der Wirbelsäule verankert sind.

Aus der US 6,440,169 B1 ist eine Stabilisierungseinrichtung für die Wirbelsäule bekannt, die einen sich zwischen Verankerungspunkten in benachbarten Wirbeln erstreckenden elastischen Körper aus einer Blattfeder aufweist, die so geformt ist, daß sie die Wand einer Öffnung bildet, wobei sich die Öffnung in der anterioren/posterioren Richtung unter Bezug auf den Körper des Patienten erstreckt und in der medialen/lateralen Richtung geschlossen ist.

Aus der WO 02/102259 A2 ist eine Stabilisierungsvorrichtung für Wirbel bekannt, die zwei Pedikelschrauben und ein Federteil umfasst, wobei das Federteil die Pedikelschrauben mit einander verbindet.

Aus der EP 0667127 A1 ist eine Vorrichtung zum Zweck der Beschränkung der Bewegung zwischen Wirbeln bekannt, die mindestens zwei Pedikelschrauben und ein elastisches Kupplungselement umfasst, wobei das Kupplungselement die Pedikelschrauben mit einander verbindet.

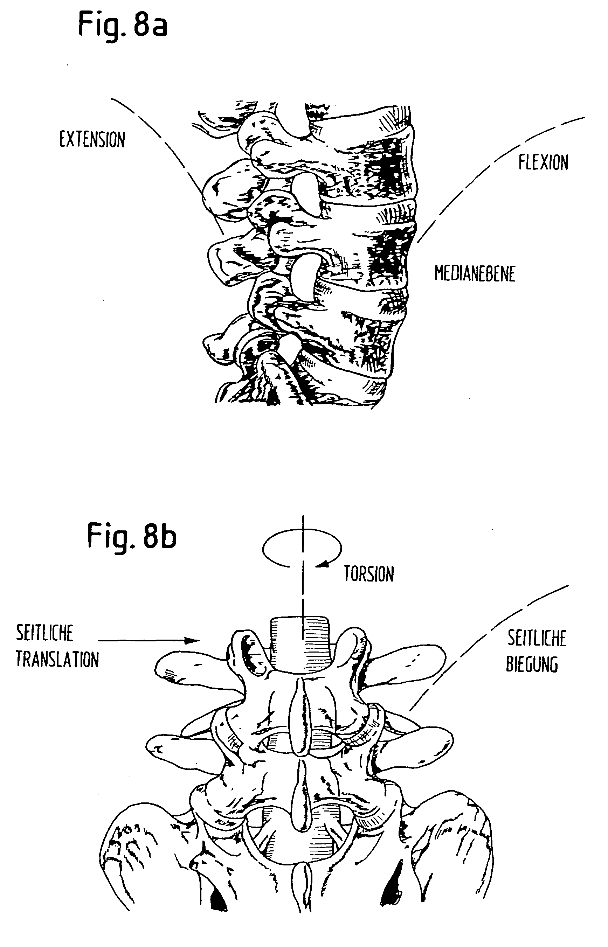

Insbesondere beim Vorhandensein einer geschädigten oder einer künstlichen Bandscheibe ist es wünschenswert, diese durch eine posteriore dynamische Stabilisierungseinrichtung hinsichtlich der zu übertragenden Kräfte in definierter Weise zu unterstützen und die Bewegung zu kontrollieren. Dabei sollen eine Flexion und eine Extension der Wirbelsäule bzw. des die betreffende Bandscheibe enthaltenden Segments, wie in Fig. 8a ersichtlich ist, möglich sein, während eine seitliche Translationsbewegung, sowie eine Torsion um die die Mittellängsachse der Wirbelsäule, wie in Fig. 8b gezeigt ist, unerwünscht sind.

Es ist Aufgabe der Erfindung eine dynamische Stabilisierungseinrichtung zur Stabilisierung und zur Bewegungsbegrenzung von benachbarten Wirbeln oder Knochen bereitzustellen, welche einfach gebaut ist und mit der eine Bewegungsbegrenzung dahingehend erzielbar ist, daß eine Flexion und Extension in begrenztem Maße erlaubt ist, eine seitliche Translationsbewegung bzw. eine Rotationsbewegung der Wirbel oder Knochen zueinander jedoch weitgehend verhindert wird.

Die Aufgabe wird durch eine Stabilisierungseinrichtung nach dem Patentanspruch 1 gelöst.

Weiterbildungen der Erfindung sind in den Unteransprüchen angegeben.

Die Erfindung weist den Vorteil auf, daß eine gewünschte Bewegungsbegrenzung auf einfache Weise durch Auswahl geeignet dimensionierter stabförmiger Elemente erzielt werden kann. Die stabförmigen Elemente der Stabilisierungseinrichtung sind ferner leicht herstellbar. Die Stabilisierungseinrichtung ist vorteilhaft anwendbar zur Stabilisierung der Wirbel bei der Verwendung von künstlichen Bandscheiben.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Draufsicht auf die erfindungsgemäße Stabilisierungseinrichtung nach einer ersten Ausführungsform von der posterioren Seite der Wirbelsäule gesehen;
- Fig. 2a: eine perspektivische Ansicht eines stabförmigen Elements der Stabilisierungseinrichtung nach Fig. 1;
- Fig. 2b: eine perspektivische Ansicht der Stabilisierungseinrichtung von Fig. 1 von oben mit bevorzugter Ausbildung der stabförmigen Elemente ohne Darstellung der oberen zwei Verankerungselemente;
- FIG. 3: eine teilgeschnittene Ansicht eines Verankerungselements zum Verankern des stabförmigen Elements von Fig. 2 nach einer ersten Ausführungsform;
- Fig. 4: eine teilgeschnittene Explosionsdarstellung eines Verankerungselements zum Verankern des stabförmigen Elements von Fig. 2 nach einer zweiten Ausführungsform;
- Fig 5: eine teilgeschnittene Ansicht des Verankerungselements von Fig. 4 in Verbindung mit dem stabförmigen Element;
- Fig. 6a: eine schematische Darstellung der Bewegungsmöglichkeit eines stabförmigen Elements der Stabilisierungseinrichtung von Fig. 1;
- Fig. 6b: eine schematische Darstellung der Funktionsweise der Stabilisierungseinrichtung nach der ersten Ausführungsform;
- Fig. 7: eine Abwandlung der Stabilisierungseinrichtung die keine Ausführungsform der Erfindung ist;
- Fig. 8a: eine Darstellung eines Abschnitts der Wirbelsäule von der Seite; und
- Fig. 8b: eine Darstellung eines Abschnitts der Wirbelsäule von hinten.

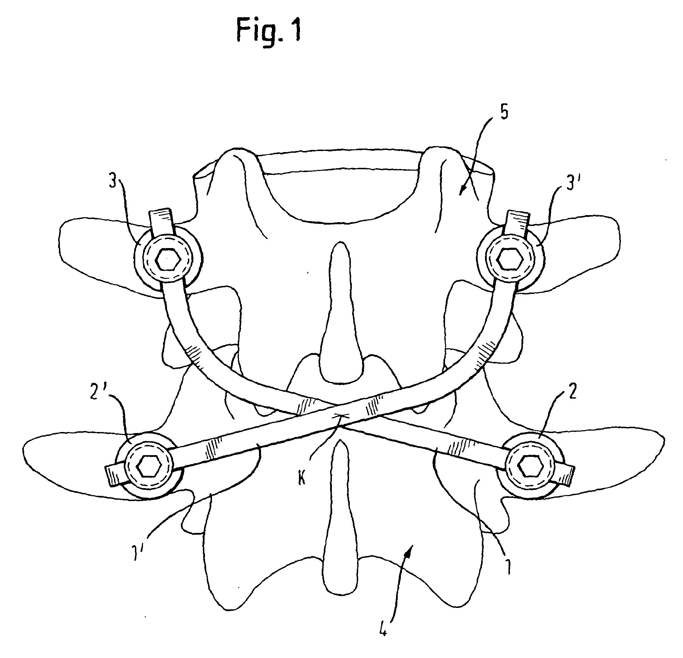

Wie aus Fig. 1 ersichtlich ist, umfaßt die Stabilisierungseinrichtung in einer ersten Ausführungsform ein erstes und ein zweites stabförmiges Element 1, 1'. Diese sind jeweils mit zwei Verankerungselementen, im gezeigten Anwendungsbeispiel mit Pedikelschrauben 2, 3 bzw. 2', 3' verbunden, welche in den Pedikeln zweier benachbarter Wirbel 4, 5 verankert sind. Die erste Pedikelschraube 2 des stabförmigen Elements 1 ist dabei im rechten Pedikel des unteren Wirbels 4 verankert, während die zweite Pedikelschraube 3 des stabförmigen Elements 1 im linken Pedikel des oberen Wirbels 5 verankert ist. Symmetrisch dazu ist die erste Pedikelschraube 2' des stabförmigen Elements 1' im linken Pedikel des unteren Wirbels 4 verankert, während die zweite Pedikelschraube 3' des stabförmigen Elements 1' im rechten Pedikel des oberen Wirbels 5 verankert ist. Somit kreuzen sich die stabförmigen Elemente 1, 1' an einem Punkt K in der Symmetrieebene.

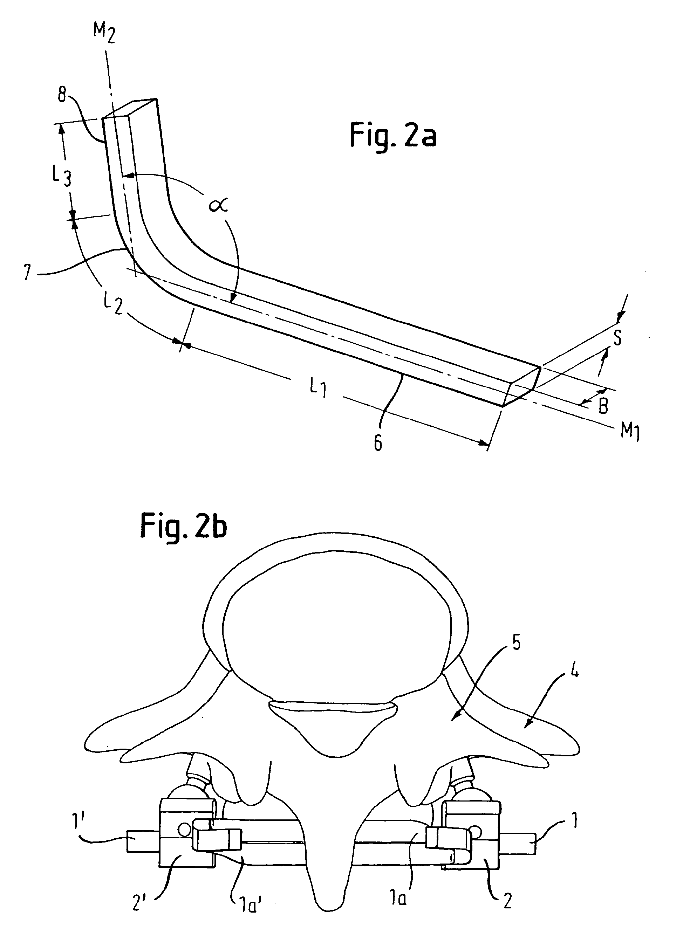

Wie aus Fig. 2a ersichtlich ist, ist das erste stabförmige Element 1 als ein gekrümmter Stab mit einem rechteckigen Querschnitt ausgebildet. Das stabförmige Element 1 weist einen ersten geraden Abschnitt 6 mit einer Länge L1, daran angrenzend einen gekrümmten Abschnitt 7 mit einer Länge L2 und daran angrenzend wieder einen geraden Abschnitt 8 mit einer Länge L3 auf. Die Mittellängsachsen M1 und M2 der geraden Abschnitte schließen in dem gezeigten Ausführungsbeispiel einen stumpfen Winkel α miteinander ein. Die Länge L1 des ersten geraden Abschnitts beträgt in dem gezeigten Ausführungsbeispiel etwa das Dreifache der Länge L3 des zweiten geraden Abschnitts 8. Die Krümmung verläuft über die breite Seite B des stabförmigen Elements. Die Biegesteifigkeit gegenüber einer Kraftkomponente, die senkrecht auf seine breite Seite B wirkt, ist kleiner als gegenüber einer Kraftkomponente, die senkrecht auf seine schmale Seite S wirkt. Ferner ist die Biegesteifigkeit des stabförmigen Elements aufgrund der Hebelwirkung durch die unterschiedlichen Längen L1 und L3 der geraden Abschnitte 6, 8 gegenüber einer Kraftkomponente, die am Ende des langen Abschnitts 6 senkrecht zum Stab angreift, geringer, als gegenüber einer Kraftkomponente, die am Ende des kurzen Abschnitts 8 senkrecht angreift.

Das stabförmige Element besitzt somit eine orientierte Biegesteifigkeit, die durch Wahl des Querschnitts, der Krümmung und der Länge in gewünschter Weise bei der Herstellung einstellbar ist. Das stabförmige Element 1 ist ferner einstückig aus einem körperfreundlichen Material, wie z.B. aus Titan ausgebildet und bevorzugt aus dem Vollen gefräßt.

Das zweite stabförmige Element 1' ist in der einfachsten Ausführungsform spiegelsymmetrisch zu dem ersten stabförmigen Element 1 befestigt, so daß die gekrümmten Abschnitte in entgegengesetzte Richtungen zeigen.

Fig. 2b zeigt die Stabilisierungseinrichtung von Fig. 1 von oben mit einer bevorzugten Ausbildung der stabförmigen Elemente 1, 1', wobei jeweils die oberen Verankerungselemente nicht dargestellt sind. Die stabförmigen Elemente 1, 1' sind in einem Bereich 1a, 1a' angrenzend an den Abschnitt mit dem sie im Verankerungselement befestigt sind, derart aus der Ebene, die durch die Verbindungsstellen definiert ist, nach außen gebogen, daß sie im Betriebszustand der Stabilisierungseinrichtung aneinander vorbeigeführt sind, ohne sich zu behindern. Dies ermöglicht, daß die Verbindungsstellen der Verankerungselemente zu den stabförmigen Elementen alle in einer Ebene senkrecht zu der in Fig. 8a angedeuteten Medianebene liegen.

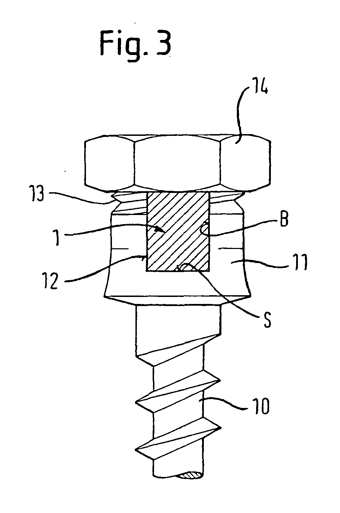

Wie in Fig. 3 gezeigt ist, ist in einer ersten Ausführungsform das stabförmige Element 1, 1' in einer monoaxialen Pedikelschraube fixiert. Diese weist einen Gewindeschaft 10 mit einem Knochengewinde und ein damit starr verbundenes Aufnahmeteil 11 auf. Das Aufnahemteil 11 ist im wesentlichen zylindrisch ausgebildet und weist eine sich von seinem dem Gewindeschaft 10 abgewandten freien Ende zum Gewindeschaft hin erstreckende Ausnehmung 12 mit rechteckigem Querschnitt auf, die so bemessen ist, daß darin das stabförmige Element 1, 1' einlegbar und noch in Richtung seiner Längsachse verschiebbar ist. Die Ausnehmung 12 ist so gebildet, daß die schmale Seite S des stabförmigen Elements 1 im Grund der Ausnehmung ruht. Ausgehend von seinem freien Ende weist das Aufnahmeteil ein Außengewinde 13 auf, das sich über eine vorbestimmte Länge erstreckt, die so bemessen ist, daß das Außengewinde bei eingelegtem stabförmigen Element bis unterhalb dessen Oberseite reicht. Zum Fixieren des stabförmigen Elements in dem Aufnahmeteil ist eine auf das Außengewinde aufschraubbare Mutter 14 vorgesehen.

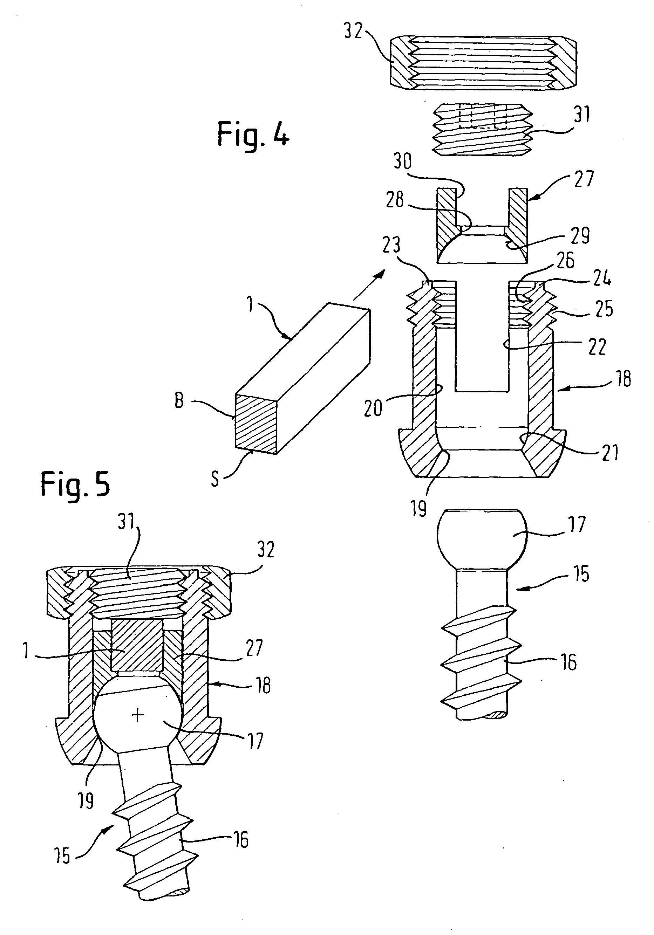

In einer weiteren Ausführungsform ist eine in den Figuren 4 und 5 gezeigte Polyaxialschraube zum Verbinden mit dem stabförmigen Element 1, 1' vorgesehen. Diese weist ein Schraubenelement 15 mit einem Gewindeschaft 16 mit einem Knochengewinde und einen kugelsegmentförmigen Kopf 17 mit einer nichtdargestellten Ausnehmung zum Einschrauben auf. Ferner ist ein mit dem Schraubenelement 15 verbindbares Aufnahmeteil 18 vorgesehen. Das Aufnahmeteil 18 ist im wesentlichen zylindrisch ausgebildet und hat an seinem einen Ende eine axialsymmetrisch ausgerichtete erste Bohrung 19, deren Durchmesser kleiner als der des Kopfes 17 ist. Ferner weist das Aufnahmeteil 18 eine koaxiale zweite Bohrung 20 auf, die auf dem der ersten Bohrung 19 gegenüberliegenden Ende offen ist und deren Durchmesser so groß ist, daß das Schraubenelement 15 durch das offene Ende mit seinem Gewindeschaft durch die erste Bohrung 19 hindurch und mit dem Kopf 17 bis zum Grund der zweiten Bohrung 20 führbar ist. Zwischen der ersten und der zweiten Bohrung ist ein sphärischer Abschnitt 21 zum Anliegen des Kopfs 17 vorgesehen.

Das Aufnahmeteil 18 weist ferner eine zur Mitte des Teiles symmetrisch angeordnete Ausnehmung 22 mit einem rechteckigen Querschnitt zur Aufnahme des stabförmigen Elements 1, 1' auf, durch die zwei freie Schenkel 23, 24 gebildet sind. In einem Bereich angrenzend an ihr freies Ende weisen die Schenkel 23, 24 in diesem Ausführungsbeispiel ein Außengewinde 25 und ein Innengewinde 26 auf.

Es ist ferner ein in der Bohrung 20 zu dem Kopf hin verschiebbares Druckelement 27 vorgesehen. Das Druckelement 27 weist eine koaxiale Bohrung 28 zum Hindurchführen eines Schraubendrehers, sowie an seiner dem Kopf 17 zugewandten Seite eine sphärische Ausnehmung 29 für den Kopf 17 auf. An seiner dem Kopf abgewandten Seite weist das Druckelement 27 eine Ausnehmung 30 mit rechteckigem Querschnitt zur Aufnahme des stabförmigen Elements 1, 1' auf. Die Breite der Ausnehmung 30 ist gerade ein wenig größer als die schmale Seite S des stabförmigen Elements 1, 1', so daß dieses mit seiner schmalen Seite S zum Grund der Ausnehmung hin einführbar ist und in der Ausnehmung in Längsrichtung verschiebbar ist. Die Tiefe der Ausnehmung 30 ist etwas kleiner als die Höhe B des stabförmigen Elements.

Zum Fixieren des stabförmigen Elements 1, 1' in dem Aufnahmeteil und zum Fixieren der Winkelstellung des Schraubenelements 15 relativ zu dem Aufnahmeteil sind ferner eine zwischen die Schenkel einschraubbare Innenschraube 31 und zur Sicherung eine auf die Schenkel aufschraubbare Mutter 32 vorgesehen.

Im Betrieb werden zunächst die Pedikelschrauben 2, 3, 2', 3' in die mit der Stabilisierungseinrichtung zu verbindenden Wirbel 4, 5 eingeschraubt. Im Fall der Monoaxialschrauben nach Fig. 4 werden die Gewindeschäfte 10 soweit eingeschraubt, bis die Aufnahmeteile 11 der mit jeweils einem stabförmigen Element 1, 1' zu verbindenden Pedikelschrauben 2, 3 bzw. 2', 3' zueinander so ausgerichtet sind, daß das stabförmige Element ohne zu verkanten einführbar ist. Dann wird das stabförmige Element durch Aufschrauben der Mutter 14 fixiert.

Im Fall der Polyaxialschrauben nach den Figuren 4 und 5 wird erst das Schraubenelement 15 in das Aufnahmeteil 18 eingeführt, dann das Druckelement 27 eingesetzt und anschließend das Schraubenelement in den Pedikel eingeschraubt. Anschließend wird das stabförmige Element 1 bzw. 1' in die Aufnahmeteile eingesetzt. Die Aufnahmeteile 18 zweier mit dem stabförmigen Element 1 bzw. 1' zu verbindender Pedikelschrauben 2, 3 bzw. 2', 3' richten sich dabei auf Grund der gelenkigen Verbindung zwischen dem Kopf 17 und dem Aufnahmeteil 18 selbst richtig zum Stab hin aus, was bei der überkreuzten Anordnung der stabförmigen Elemente von Vorteil ist. Anschließend werden die Aufnahmeteile relativ zum Kopf und zum Stab durch die Innenschraube und die Mutter fixiert.

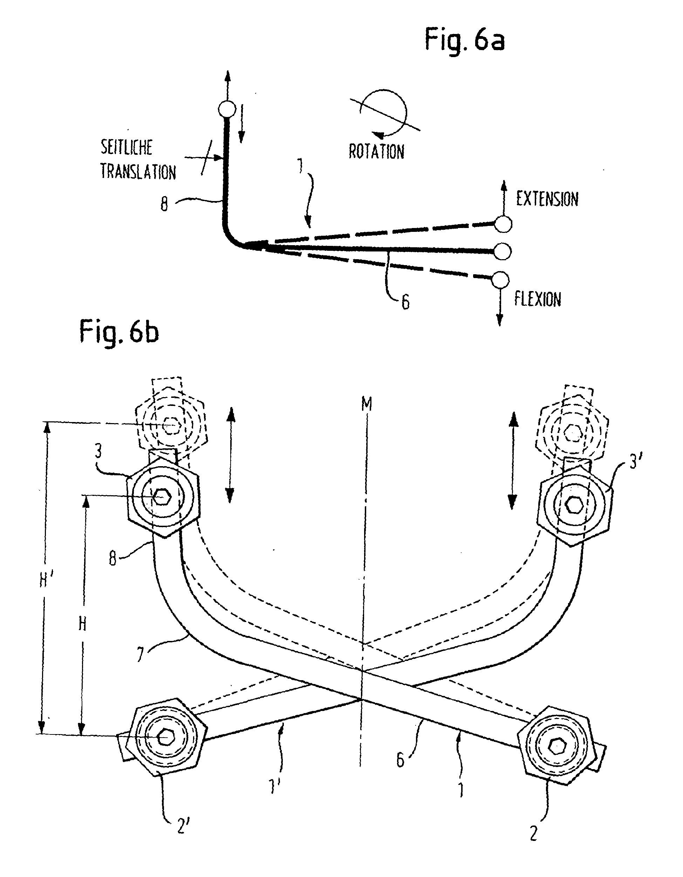

Die Figuren 6a und 6b zeigen schematisch die Funktionsweise der Stabilisierungseinrichtung. Wie in Fig. 6a gezeigt ist kann das stabförmige Element 1 in unbelastetem Zustand in idealisierter Weise als ein Winkelhebel mit einem langen Hebelarm 6 und einem kurzen Hebelarm 8 angesehen werden. Bei Belastung durch Flexion oder Extension des Wirbelsäulensegments führt eine kleine an den Verankerungspunkten senkrecht zum langen Hebelarm 6 angreifende Kraftkomponente zur elastischen Deformation des stabförmigen Elements 1, indem der lange Hebelarm ausgelenkt wird und sich der Krümmungsradius relativ zu der Ruhestellung bei der Flexion vergrößert und bei der Extension verkleinert.

In Fig. 6b zeigen die durchgezogenen Linien die stabförmigen Elemente 1, 1' in einem ersten Zustand und die gestrichelten Linien in einem zweiten Zustand, der in diesem Fall eine Flexion des Wirbelsäulensegments ist. In dem ersten Zustand haben die Pedikelschrauben 2', 3 mit denen das stabförmige Element 1 verbunden ist, in Richtung der Mittellängsachse M der Wirbelsäule einen Abstand H voneinander. Bei der Flexion des Wirbelsäulensegments kommt es zu einer Biegung des stabförmigen Elements 1, wobei der Krümmungsradius dabei größer wird und somit in dem gezeigten zweiten Zustand der Abstand der Pedikelschrauben in Richtung der Mittellängsachse auf H' vergrößert wird. Umgekehrt wird bei einer Extension des Wirbelsäulensegments der Krümmungsradius der stabförmigen Elemente verkleinert. Damit verringert sich der Abstand der Pedikelschrauben.

Bei Einwirkung einer Kraftkomponente senkrecht zu dem kurzen Hebelarm, ist eine Änderung der Krümmung der stabförmigen Elemente aufgrund der erforderlichen größeren Kraft erschwert oder gar nicht möglich. Somit wird eine seitliche Translationsbewegung verhindert. Aufgrund der länglichen Ausbildung der stabförmigen Elemente besteht ferner weitgehend Rotationsstabilität gegenüber einer Rotationsbewegung der Wirbel zueinander.

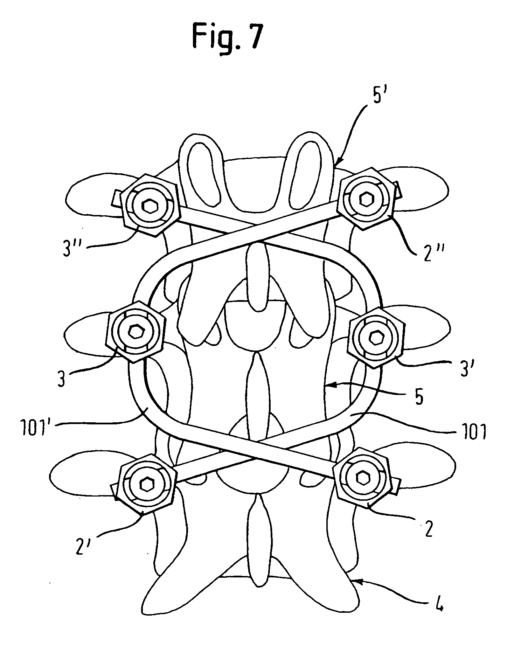

Fig. 7 zeigt eine Abwandlung der Stabilisierungseinrichtung. Diese Stabilisierungseinrichtung erstreckt sich auf zwei Bewegungssegmente, bestehend jeweils aus den Wirbeln 4, 5 bzw. 5, 5'. Es sind stabförmige Elemente 101, 101' vorgesehen, die eine Form haben, die durch Spiegelung eines stabförmigen Elements 1, 1' der ersten Ausführungsform an einer auf dem freien Ende des kurzen Abschnitts senkrecht stehenden Ebene entsteht. Die stabförmigen Elemente 101, 101' sind jeweils durch Pedikelschrauben 2, 2'' bzw. 2', 3'' an ihren Enden in den Pedikeln auf derselben Seite der jeweils übernächsten Wirbel 4, 5' verankert und mit ihrem mittleren Abschnitt über Pedikelschrauben 3 bzw. 3' in den Pedikeln der gegenüberliegenden Seite des dazwischenliegenden Wirbels 5 verankert.

Eine mehrsegmentale Ausbildung ist ebenso möglich.

Abwandlungen der zuvor beschriebenen Ausführungsformen sind möglich. So werden die Längen der einzelnen Abschnitte der stabförmigen Elemente entsprechend den Dimensionen der zu verbindenden Wirbel gewählt. Die Erfindung ist nicht darauf beschränkt, daß die stabförmigen Elemente der Stabilisierungseinrichtung der ersten Ausführungsform einen langen und einen kurzen geraden Abschnitt aufweisen. Es können auch nur ein langer und ein gekrümmter Abschnitt vorhanden sein. Bei der Stabilisierungseinrichtung nach Fig. 7 können die geraden Abschnitte auch unterschiedlich lang sein, entscheidend ist, daß das stabförmige Element zwischen dem einen Befestigungspunkt und dem anderen Befestigungspunkt eine Krümmung aufweist, damit das stabförmige Element unter Einwirkung von Biegemomenten eine Abstandsänderung der Befestigungspunkte bewirkt.

Ferner sind die Merkmale der beschriebenen Ausführungsformen miteinander kombinierbar. Die Stabilisierungseinrichtung der ersten Ausführungsform kann auch stabförmige Elemente mit kreisförmigem Querschnitt aufweisen. Auch ein quadratischer Stabquerschnitt ist möglich, wobei dann aber ein Freiheitsgrad bei der Wahl der biegeelastischen Eigenschaften des stabförmigen Elements wegfällt.

Anstelle der beschriebenen Monoaxialschrauben und Polyaxialschrauben können auch Monoaxialschrauben und Polyaxialschrauben verwendet werden, bei denen die Kopf- oder die Stabfixierung auf andere Weise erfolgt.

Die Erfindung ist ferner nicht auf eine Stabilisierungseinrichtung für die Wirbelsäule beschränkt, sondern kann auch zum Stabilisieren von Knochen eingesetzt werden.

## Patentansprüche

1. Stabilisierungseinrichtung zur dynamischen Stabilisierung von Wirbeln oder Knochen, mit
einem ersten Knochenverankerungselement (2) und einem zweiten Knochenverankerungselement (2') zur Verankerung in einem ersten Knochenteil (4) oder einem Wirbel und mit
einem dritten Knochenverankerungselement (3) und einem vierten Knochenverankerungselement (3') zur Verankerung in einem zweiten Knochenteil (5) oder Wirbel,
einem ersten stabförmigen Element (1; 101), das mit dem ersten oder mit dem zweiten Knochenverankerungselement (2) und mit dem dritten oder dem vierten Knochenverankerungselement verbunden ist;
einem zweiten stabförmigen Element (1'; 101'), das mit dem zweiten oder mit dem ersten Knochenvorankerungselement und mit dem vierten oder dem dritten Knochenverankerungselement verbunden ist;
wobei die stabförmigen Elemente (1, 1' ; 101; 101') zwischen den Verbindungsstellen jeweils einen gekrümmten Abschnitt (7) aufweisen, der sich unter Einwirkung einer über ein Verankerungselement auf das stabförmige Element ausgeübten Kraft elastisch verformt; **dadurch gekennzeichnet, dass** die stabförmigen Elemente im wesentlichen L-förmig gekrümmt sind.

2. Stabilisierungseinrichtung nach Anspruch 1, **dadurch gekennzeichnet, daß** die stabförmigen Elemente (1, 1'; 101, 101') so angeordnet sind, daß sie sich wenigstens einmal überkreuzen.

3. Stabilisierungseinrichtung nach Anspruch 1 oder 2
wobei die stabförmigen Elemente und die Verankerungselemente im Betriebszustand so angeordnet sind, daß die Deformation unter Einwirkung einer Kraft in einer Hauptbewegungsrichtung der Knochenteile oder Wirbel relativ zueinander erfolgt, während eine Deformation unter Krafteinwirkung in einer Richtung im wesentlichen senkrecht zu der Hauptbewegungsrichtung unterdrückt ist.

4. Stabilisierungseinrichtung nach einem der Ansprüche 1 bis 3, **dadurch gekennzeichnet, daß** das erste (2) und das dritte Knochenverankerungselement (3) relativ zur Hauptbewegungsrichtung diagonal gegenüberliegend angeordnet sind.

5. Stabilisierungseinrichtung nach einem der Ansprüche 1 bis 4, **dadurch gekennzeichnet, daß** die Stabilisierungseinrichtung zum Stabilisieren wenigstens zweier benachbarter Wirbel (4, 5) vorgesehen ist und so ausgebildet ist, daß die mit einem stabförmigen Element verbundenen Knochenverankerunqselemente je in diagonal gegenüberliegenden Pedikeln der Wirbel verankerbar sind.

6. Stabilisierungseinrichtung nach einem der Ansprüche 1 bis 5, **dadurch gekennzeichnet, daß** das zweite (2') und das vierte (3') Knochenverankerungselement und das diese verbindende zweite stabförmige Element ( 1' ; 101') symmetrisch zu dem ersten (2) und dem dritten (3) Knochenverankerungselement mit dem ersten stabförmigen Element ( 1; 101) angeordnet sind.

7. Stabilisierungseinrichtung nach einem der Ansprüche 1 bis 6, **dadurch gekennzeichnet**, die stabförmigen Elemente einen rechteckigen oder quadratischen Querschnitt aufweisen.

8. Stabilisierungseinrichtung nach einem der Ansprüche 1 bis 6, **dadurch gekennzeichnet, daß** die stabförmigen Elemente einen kreisförmigen Querschnitt aufweisen.

9. Stabilisierungseinrichtung nach einem der Ansprüche 1 bis 8, **dadurch gekennzeichnet, daß** das Verankerungselement je einen Verankerungsabschnitt (10; 15) zum Verankern in einem Knochen und ein mit diesem verbundenes Aufnahmeteil (11; 18) aufweist, welches eine dem Querschnitt des jeweiligen stabförmigen Elements angepaßte Ausnehmung (12; 22) zur Aufnahme des stabförmigen Elements hat.

10. Stabilisierungseinrichtung nach Anspruch 9, **dadurch gekennzeichnet, daß** der Verankerungsabschnitt (10) monoaxial mit dem Aufnahmeteil (11) verbunden ist.

11. Stabilisierungseinrichtung nach Anspruch 9, **dadurch gekennzeichnet, daß** der Verankerungsabschnitt (15) polyaxial mit dem Aufnahmeteil (18) verbunden ist.

12. Stabilisierungseinrichtung nach einem der Ansprüche 1 bis 11, **dadurch gekennzeichnet, daß** die Anzahl der Verankerungselemente ein Vielfaches von zwei beträgt und die stabförmigen Elemente (101, 101') jeweils mehr als zwei Verankerungselemente verbinden.

## Claims

1. Stabilization device for the dynamic stabilization of vertebrae or bones, comprising:
a first bone anchoring element (2) and a second bone anchoring element (2') for anchoring in a first bone part (4) or a vertebra and
a third bone anchoring element (3) and a fourth bone anchoring element (3') for anchoring in a second bone part (5) or vertebra,
a first rod-shaped element (1; 101) being connected with the first or with the second bone anchoring element (2) and with the third or the fourth bone anchoring element;
a second rod-shaped element (1'; 101') being connected with the second or with the first bone anchoring element and with the fourth or the third bone anchoring element;
wherein the rod-shaped elements (1, 1'; 101; 101') comprise between the points of connection a curved section (7), respectively, which elastically deforms under the action of a force acting via the anchoring element onto the rod-shaped element; **characterized in that**
the rod-shaped elements are substantially curved in a L-shape.

2. Stabilization device according to claim 1, wherein the rod-shaped elements (1, 1'; 101, 101') are arranged in such a way that they cross each other at least once.

3. Stabilization device according to claim 1 or 2, wherein the rod-shaped elements and the anchoring elements are arranged in the operating condition in such a way that the deformation under action of a force occurs in a main motion direction of the bone parts or vertebrae relative to each other, while a deformation under action of a force in a direction substantially perpendicular to the main motion direction is suppressed.

4. Stabilization device according to one of claims 1 to 3, wherein the first (2) and the third bone anchoring element (3) are arranged diagonally opposite relative to the main motion direction.

5. Stabilization device according to one of claims 1 to 4, wherein the stabilization device is provided for stabilizing at least two neighboring vertebrae (4, 5) and is configured in such a way that the bone anchoring elements connected to a rod-shaped element are anchorable in diagonally opposite pedicles of the vertebrae.

6. Stabilization device according to one of claims 1 to 5, wherein the second (2') and the fourth (3') bone anchoring element and the second rod-shaped element (1'; 101') connecting these are arranged symmetrically to the first (2) and the third (3) bone anchoring element with the first rod-shaped element (1; 101).

7. Stabilization device according to one of claims 1 to 6, wherein the rod-shaped elements have a rectangular or square cross section.

8. Stabilization device according to one of claims 1 to 6, wherein the rod-shaped elements have a circular cross section.

9. Stabilization device according to one of claims 1 to 8, wherein the anchoring element comprises an anchoring section (10; 15) for anchoring in a bone and a receiving part (11; 18) connected thereto, respectively, which has a recess (12; 22) adapted to the cross section of the respective rod-shaped element for receiving the rod-shaped element.

10. Stabilization device according to claim 9, wherein the anchoring section (10) is connected monoaxially to the receiving part (11).

11. Stabilization device according to claim 9, wherein the anchoring section (15) is connected polyaxially to the receiving part (18).

12. Stabilization device according to one of claims 1 to 11, wherein the number of anchoring elements is a multiple of two and the rod-shaped elements (101, 101') connect more than two anchoring elements.

## Revendications

1. Dispositif de stabilisation, pour la stabilisation dynamique du rachis ou des os, avec :
un premier élément d'ancrage d'os (2) et un deuxième élément d'ancrage dans l'os (2'), pour l'ancrage dans une première partie d'os (4) ou une vertèbre, et avec
un troisième élément d'ancrage d'os (3) et un quatrième élément d'ancrage dans l'os (3'), pour l'ancrage dans une deuxième partie d'os (5) ou une vertèbre,
un premier élément en forme de barre (1 ; 101), relié au premier ou au deuxième élément d'ancrage dans l'os (2) et au troisième ou au quatrième élément d'ancrage dans l'os
un deuxième élément en forme de barre (1' ; 101'). relié au deuxième ou au premier élément d'ancrage dans l'os (2) et au quatrième ou au troisième élément d'ancrage dans l'os ;
les éléments en forme de barre (1. 1' ; 101 ; 101') présentant chacun, entre les points de liaison, un tronçon (7) incurvé, se déformant élastiquement sous l'effet d'une force exercée sur l'élément en forme de barre ;
**caractérisé en ce que** les éléments en forme de barre sont incurvés sensiblement en forme de L.

2. Dispositif de stabilisation selon la revendication 1, **caractérisé en ce que** les éléments en forme de barre (1, 1' 101 ; 101') sont disposés de manière à se croiser au moins une fois.

3. Dispositif de stabilisation selon la revendication 1 ou 2, **caractérisé en ce que** les éléments en forme de barre et les éléments d'ancrage, en état de fonctionnement sont disposés de manière que la déformation sous l'effet d'une force se fasse, les uns par rapport aux autres, dans une direction de déplacement principale des parties d'os ou vertèbres, tandis qu'une déformation sous l'effet d'une force, dans une direction sensiblement perpendiculaire à la direction de déplacement principale, soit empêchée.

4. Dispositif de stabilisation selon l'une des revendications 1 à 3, **caractérisé en ce que** le premier (2) et le troisième (3) élément d'ancrage dans l'os sont disposés de façon diagonalement opposée par rapport à la direction de déplacement principale.

5. Dispositif de stabilisation selon l'une des revendications 1 à 4, **caractérisé en ce que** le dispositif de stabilisation est prévu pour la stabilisation d'au moins deux vertèbre (4, 5) voisines, et est réalisé de manière que les éléments d'ancrage dans l'os, reliés à un élément en forme de barre, soient chacun susceptibles d'être ancrés dans des pédicules, diagonalement opposés, des vertèbres.

6. Dispositif de stabilisation selon l'une des revendications 1 à 5, **caractérisé en ce que** le deuxième (2') et le quatrième (3') élément d'ancrage dans l'os, et le deuxième élément en forme de barre (1' ; 10') reliant ceux-ci, sont disposés symétriquement par rapport au premier (2) et au troisième (3) élément d'ancrage dans l'os, avec le premier élément en forme de barre (1 ; 101).

7. Dispositif de stabilisation selon l'une des revendications 1 à 6, **caractérisé en ce que** les éléments en forme de barre présentent une section transversale rectangulaire ou carrée.

8. Dispositif de stabilisation selon l'une des revendications 1 à 7, **caractérisé en ce que** les éléments en forme de barre présentent une section transversale en forme cercle.

9. Dispositif de stabilisation selon l'une des revendications 1 à 8, **caractérisé en ce que** l'élément d'ancrage présente chaque fois un tronçon d'ancrage (10 ; 15) pour l'ancrage dans un os, et une partie de logement (11 ; 18) reliée à celui-ci, comprenant un évidemment (12 ; 22), adapté à la section transversale de l'élément en forme de barre respectif, afin de loger l'élément en forme de barre.

10. Dispositif de stabilisation selon la revendication 9, **caractérisé en ce que** le tronçon d'ancrage (10) est relié de façon monoaxiale à la partie de logement (11).

11. Dispositif de stabilisation selon la revendication 9, **caractérisé en ce que** le tronçon d'ancrage (15) est relié de façon polyaxiale à la partie de logement (18).

12. Dispositif de stabilisation selon l'une des revendications 1 à 11, **caractérisé en ce que** le nombre des éléments d'ancrage est un multiple de deux, et les éléments en forme de barre (101, 101' ) relient chacun plus de deux éléments d'ancrage.
